# EUROPÄISCHE PATENTSCHRIFT

(11) **EP 2 632 909 B1**
(45) Veröffentlichungstag und Bekanntmachung des Hinweises auf die Patenterteilung: **25.02.2015**
(21) Anmeldenummer: 11776413.4
(22) Anmeldetag: 26.10.2011
(51) Int. Cl.: C07D 295/088

(54) **VERFAHREN ZUR HERSTELLUNG VON 1,4-BISHYDROXYETHYL-PIPERAZIN**
PROCESS FOR PREPARING 1,4-BISHYDROXYETHYLPIPERAZINE
PROCÉDÉ DE PRÉPARATION DE 1,4-BISHYDROXYÉTHYL-PIPÉRAZINE

(30) Priorität: 29.10.2010 EP 10189475
(43) Veröffentlichungstag der Anmeldung: 04.09.2013
(73) Patentinhaber: BASF SE, 67056 Ludwigshafen (DE)
(72) Erfinder: WIGBERS, Christof Wilhelm, 68167 Mannheim (DE); CHALLAND, Nina, 68165 Mannheim (DE); MELDER, Johann-Peter, 67459 Böhl-Iggelheim (DE); RHEUDE, Udo, 67166 Otterstadt (DE); DOSTALEK, Roman, 67271 Neuleiningen (DE)
(86) Internationale Anmeldenummer: PCT/EP2011/068700
(87) Internationale Veröffentlichungsnummer: WO 2012/055893

(56) Entgegenhaltungen:
- DATABASE WPI Week 198731 Thomson Scientific, London, GB; AN 1987-218358 XP002664153, & JP 62 145076 A (KAO CORP) 29. Juni 1987 (1987-06-29) in der Anmeldung erwähnt

## Beschreibung

Die vorliegende Anmeldung schließt durch Verweis die am 29. Oktober 2010 eingereichte vorläufige US-Anmeldung Nr. 61/407,936 ein.

Die vorliegende Erfindung betrifft ein Verfahren zur Herstellung von 1,4-Bishydroxyethylpiperazin (BHEPIP) der Formel I

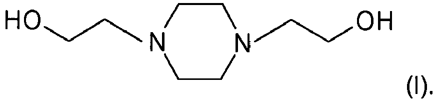

1,4-Bis(2-hydroxyethyl)piperazin I kann für die Herstellung von Pharmazeutika (vgl. z. B. DE 2706826 A), von Tensiden, als Katalysator für die Herstellung von Polyurethanen (vgl. z. B. JP 62145076 A, Kao Corp.) und als Ausgangsverbindung für die Herstellung von Diazabicyclo(2.2.2)oktan (DABCO) (vgl. z. B. US 3,166,558 (1965), Air Products and Chem.) verwendet werden.

1,4-Bishydroxyethylpiperazin I wurde erstmalig durch Umsetzung von Piperazin (PIP) mit 2-Chlorethanol (J. Chem. Soc. 93, Seite 1802 (1908)) und später auch aus Piperazin und Ethylenoxid (DE 1 954 546 A (1971), BASF AG) hergestellt.

Spätere Untersuchungen ergaben, dass sich BHEPIP (I) nicht nur aus Piperazin, sondern auch aus Dietha¬nolamin (DEOA) herstellen lässt. Diethanolamin fällt bei der Herstellung von Ethanolamin aus Ethylenoxid und Ammoniak als Nebenprodukt an.

So lässt sich BHEPIP (I) durch Erhitzen von Diethanolamin in Gegenwart von Mono- oder Dicarbonsäuren gewinnen (DE 917 784 (1954), Henkel & Cie GmbH). In Beispiel 1 der DE 917 784 wurde ein Gemisch aus Diethanolamin und Eisessig (Molverhältnis 1 : 0,1) unter Rühren und Durchleiten von Stickstoff und Kondensieren von Reaktionswasser 20 Stunden auf 200 °C erhitzt. Die Ausbeute an I betrug 70 %.

Aus J. Am. Chem. Soc. 61, 532 (1939) ist bekannt, Lösungen von Diethanolamin in Dioxan in Gegenwart von Kupferchromit-Katalysatoren einer thermischen Behandlung bei 250 bis 275 °C zu unterwerfen. Die unter Hydrierbedingungen erzielten Ausbeuten an BHEPIP (I) sind jedoch nicht höher als 50 % (vgl. DE 941 909 (1956), Henkel & Cie GmbH; Seite 2, Spalte 1, Zeile 26, bis Spalte 2, Zeile 1).

Die niedrige Ausbeute an BHEPIP (I) bei Verwendung von Kupferchromit ließ sich durch den Einsatz von Katalysatoren, die 48 % Kupferoxid, 47 % Chromoxid, 2,5 % Mangan(IV)oxid und 2,5 % Bariumoxid enthielten (JP 62145076 A, (1987), Kao Corp), auf 68 % steigern (dort Beispiel 1). Dabei wurde der Katalysator in Gegenwart eines Lösungsmittels in Suspension eingesetzt.

Nachteilig an der Verwendung der genannten Kupfer-Katalysatoren ist die Toxizität des Katalysatorbestandteils Chrom. Daher muss bei der Abtrennung, Regenerierung und/oder Entsorgung dieser Katalysatoren ein hoher Sicherheitsaufwand getrieben werden.

Die älteren EP-Patentanmeldungen mit dem Aktenzeichen 10166017.3 vom 15.06.2010 bzw. dem Aktenzeichen 10187557.3 vom 14.10.2010 (beide BASF SE) betreffen Verfahren zur Herstellung eines zyklischen tertiären Amins der Formel I 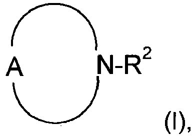 in der A eine C₄-Alkylengruppe, eine C₅-Alkylengruppe oder eine -(CH₂)₂-B-(CH₂)₂-Gruppe bedeutet, wobei B Sauerstoff (O) oder einen Rest N-R¹ bedeutet und R¹ C₁- bis C₅-Alkyl, Aryl oder C₅- bis C₇-Cycloalkyl bedeutet, und der Rest R² Methyl bzw. lineares oder verzweigtes C₂- bis C₁₆-Alkyl, C₅- bis C₇-Cycloalkyl oder C₇- bis C₂₀-Aralkyl bedeutet.

Der vorliegenden Erfindung lag die Aufgabe zugrunde, Nachteilen des Stands der Technik abzuhelfen und ein verbessertes wirtschaftliches Verfahren zur Herstellung eines zyklischen tertiären Amins aufzufinden. Insbesondere soll das Verfahren hohe Ausbeuten, Raum-Zeit-Ausbeuten (RZA) und Selektivitäten ermöglichen und ohne den Katalysatorbestandteil Chrom auskommen.

[Raum-Zeit-Ausbeuten werden angegeben in ,Produktmenge / (Katalysatorvolumen ● Zeit)' (kg/(I_{Kat}. ● h)) und/oder, Produktmenge / (Reaktorvolumen ● Zeit)' (kg/(I_{Reakto}r ● h)].

Demgemäß wurde ein Verfahren zur Herstellung von 1,4-Bishydroxyethyl-piperazin (BHEPIP) der Formel I 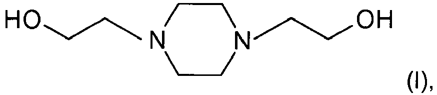 gefunden, welches dadurch gekennzeichnet ist, dass man Diethanolamin (DEOA) der Formel II 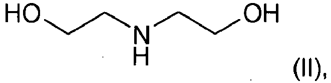 in einem Reaktor bei einer Temperatur im Bereich von 130 bis 300 °C in Gegenwart eines kupferhaltigen, chromfreien Heterogenkatalysators in der Flüssigphase umsetzt.

Die erfindungsgemäße Dimerisierung lässt sich durch die folgende Formelgleichung beschreiben:

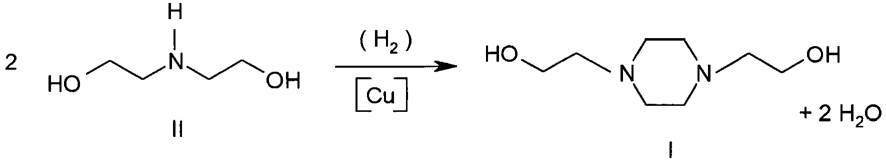

Das erfindungsgemäße Verfahren ermöglicht es, die Synthese von BHEPIP (I) in einem Reaktionsschritt durchzuführen. Dabei wird der Aminoalkohol II mit hohen Umsätzen und Ausbeuten zum zyklischen tertiären Amin I umgesetzt. Eine Rückführung von unvollständig alkylierten oder zyklisierten Zwischenprodukten ist daher im Allgemeinen nicht notwendig. Sie ist aber möglich.

Die Reaktionstemperaturfür die Herstellung von BHEPIP (I) beträgt 130 bis 300 °C, bevorzugt 150 bis 250 °C, besonders bevorzugt 170 bis 230 °C.

Nach der Stöchiometrie der Dimerisierung muss kein Wasserstoff zugeführt werden. Demgemäß wird in einer Verfahrensausgestaltung im Reaktionsschritt (i) dem Reaktor kein Wasserstoff (H₂) zugeführt.
Um die Aktivität des Hydrierkatalysators über sehr lange Zeiten konstant zu halten, ist es jedoch vorteilhaft, dem Reaktionsgemisch kontinuierlich oder von Zeit zu Zeit Wasserstoff zuzuführen.

Der Reaktionsdruck im Reaktor setzt sich bei der jeweiligen Reaktionstemperatur aus den Partialdrücken der Einsatzstoffe und der Reaktionsprodukte, gegebenenfalls von Lösungsmittel und zugeführtem Wasserstoff zusammen. Durch Aufpressen von Wasserstoff wird der Druck auf den gewünschten Reaktionsdruck erhöht.

Der Gesamtdruck (absolut) beträgt bevorzugt 10 bis 200 bar, bevorzugt 30 bis 150 bar, besonders bevorzugt 50 bis 120 bar.

Falls Wasserstoff eingesetzt wird, beträgt der Wasserstoffpartialdruck besonders 0,01 bis 130 bar, bevorzugt 0,1 bis 100 bar, besonders bevorzugt 1 bis 80 bar.

Wird die erfindungsgemäße Umsetzung kontinuierlich durchgeführt, so beträgt die Raum-ZeitAusbeute besonders 0,01 bis 5 kg/(I_{Kat.} ● h), bevorzugt 0,05 bis 3 kg/(I_{Kat.} ● h), besonders bevorzugt 0,1 bis 1,0 kg/(I_{Kat.} ● h). (I_{Kat.} = Katalysatorschüttvolumen)

Die erfindungsgemäße Umsetzung kann ohne den Zusatz eines Lösungsmittels durchgeführt werden.

Es kann jedoch vorteilhaft sein, zusätzlich ein (unter den Reaktionsbedingungen inertes) Lösungsmittel mit zu verwenden. Hierfür kommen besonders aliphatische, cycloaliphatische oder aromatische Lösungsmittel in Frage. Beispiele hierfür sind n-Hexan, n-Octan, Cyclohexan, Methylcyclohexan, Toluol, o-, m- oder p-Xylol, Tetrahydrofuran, Dioxan, Methyl-tert.-butylether oder Gemische dieser Verbindungen.

Das Gemisch aus Diethanolamin (II) und Lösungsmittel kann 5 bis 90 Gew.-%, bevorzugt 10 bis 80 Gew.-%, besonders bevorzugt 20 bis 60 Gew.-%, Lösungsmittel enthalten.

Die erfindungsgemäße Umsetzung kann diskontinuierlich oder kontinuierlich durchgeführt werden. Als Reaktoren kommen z. B. Rührreaktoren oder Rohrreaktoren in Frage. Dabei kann der Katalysator suspendiert oder bevorzugt fest angeordnet vorliegen. Festbettreaktoren können in Sumpf- oder Rieselfahrweise betrieben werden.

Als Katalysatoren eignen sich prinzipiell Hydrierkatalysatoren, bevorzugt kupferhaltige, heterogene Katalysatoren.

Prinzipiell eignet sich eine Vielzahl von chromfreien, kupferhaltigen, bevorzugt überwiegend Kupfer enthaltenen, Katalysatoren, die zusätzlich wenigstens ein weiteres Element der I., II., III., IV. oder V. Hauptgruppe, der I., II., IV., V., VII. oder VIII. Nebengruppe sowie der Lanthanide enthalten können (IUPAC: Gruppen 1 bis 15 und die Lanthanide), insbesondere Ca, Mg, Al, La, Ti, Zr, Mo, W, Mn, Ni, Co, Zn und Kombinationen davon.

Eine spezielle Ausführungsform von vorteilhaften Katalysatoren sind Raney-Katalysatoren (®), speziell Raney-Kupfer sowie kupferhaltige Metalllegierungen in Form eines Raney-Katalysators. Bevorzugt sind Raney-Katalysatoren, deren Metallkomponente zu wenigstens 95 Gew.-%, insbesondere zu wenigstens 99 Gew.-%, aus Kupfer besteht. Raney-Kupfer kann in an sich bekannter Weise durch Behandeln von Kupfer-Aluminium-Legierungen mit Alkalimetallhydroxiden hergestellt werden.

Eine weitere spezielle Ausführungsform von Katalysatoren, die sich besonders vorteilhaft für einen Einsatz eignen, sind Katalysatoren, die Kupfer in oxidischer Form sowie gegebenenfalls zusätzlich in elementarer Form enthalten.

Geeignet sind z. B. Katalysatoren, die auf einem Träger aus Kieselsäure Nickel und Kupfer neben anderen Metallen als aktive Bestandteile enthalten. Solche Katalysatoren werden z. B. in DE 26 28 087 A beschrieben. Die aktive Masse dieser Katalysatoren enthält speziell 40 bis 80 Gew.-% Nickel, 10 bis 50 Gew.-% Kupfer und 2 bis 10 Gew.-% Mangan. EP 434 062 A beschreibt geeignete Katalysatoren, die durch Reduktion eines Precursors aus Oxiden des Kupfers, Aluminiums und wenigstens eines weiteren Metalls, ausgewählt unter Magnesium, Zink, Titan, Zirkon, Zinn, Nickel und Kobalt, erhältlich sind. Aus der DE 40 21 230 A sind Kupfer-Zirkonoxid-Katalysatoren bekannt, wobei das Verhältnis von Kupferatomen zu Zirkoniumatomen, ausgedrückt als Gewichtsverhältnis, 1 : 9 bis 9 : 1 beträgt. DE 4 028 295 A beschreibt geeignete Kupfer-Mangan-Hydrierkatalysatoren. EP 552 463 A beschreibt geeignete Katalysatoren, wobei die oxidischer Form im Wesentlichen der Zusammensetzung CuₐAl_{b}Zr_{c}Mn_{d}Oₓ entspricht, wobei die folgenden Beziehungen gelten: a > 0; b > 0; c ≥ 0; d > 0; a > b/2; b > a/4; a > c; a > d; und x die zur Wahrung der Elektroneutralität pro Formeleinheit erforderliche Anzahl von Sauerstoffionen bezeichnet. EP 552 463 A beschreibt auch geeignete Katalysatoren mit einem geringeren Anteil an Aluminiumoxid. Der Katalysator nach dieser Ausführungsform entspricht im Wesentlichen der Zusammensetzung CuₐAl_{b}Zr_{c}Mn_{d}Oₓ, wobei die folgenden Beziehungen gelten: a > 0; a/40 ≤ b ≤ a/4; c ≥ 0; d > 0; a > c; 0,5d ≤ a ≤ 0,95d und x, die Wahrung der Elektroneutralität pro Formeleinheit erforderliche Anzahl von Sauerstoffionen bezeichnet. WO 2006/005505 A beschreibt Katalysatorformkörper, die sich für den Einsatz in dem erfindungsgemäßen Verfahren besonders eignen. In einer bevorzugten Ausführung umfasst das oxidische Katalysatormaterial
(a) Kupferoxid mit einem Anteil im Bereich von 50 ≤ x ≤ 80 Gew.-%, bevorzugt 55 ≤ x ≤ 75 Gew.-%,
(b) Aluminiumoxid mit einem Anteil im Bereich von 15 ≤ y ≤ 35 Gew.-%, bevorzugt 20 ≤ y ≤ 30 Gew.-%, und
(c) mindestens eines der Oxide des Lanthans, Wolframs, Molybdäns, Titans oder Zirkoniums, bevorzugt des Lanthans und/oder Wolframs, mit einem Anteil im Bereich von 2 ≤ z ≤ 20 Gew.-%, bevorzugt 3 ≤ z ≤ 15 Gew.-%,
jeweils bezogen auf das Gesamtgewicht des oxidischen Materials nach Calcinierung, wobei gilt: 80 ≤ x + y + z ≤ 100, besonders 95 ≤ x + y + z ≤ 100.

Bevorzugte Katalysatoren umfassen die folgenden Metalle in oxidischer Form, reduzierter Form (elementarer Form) oder einer Kombination davon. Metalle, die in mehr als einer Oxidationsstufe stabil sind, können vollständig in einer der Oxidationsstufen oder in verschiedenen Oxidationsstufen eingesetzt werden:
- Cu
- Cu, Ti
- Cu, Zr
- Cu, Mn
- Cu, Al
- Cu, Ni, Mn
- Cu, Al, wenigstens ein weiteres Metall, ausgewählt unter La, W, Mo, Mn, Zn, Ti, Zr, Sn, Ni, Co
- Cu, Zn, Zr
- Cu, Al, Mn, gegebenenfalls Zr.

Als inertes Trägermaterial für die erfindungsgemäßen Katalysatoren können praktisch alle Trägermaterialien des Stands der Technik, wie sie vorteilhaft bei der Herstellung von geträgerten Katalysatoren Verwendung finden, beispielsweise SiO₂ (Quarz), Porzellan, Magnesiumoxid, Zinndioxid, Siliziumcarbid, Zirkoniumdioxid (ZrO₂), Titandioxid (TiO₂) (Rutil, Anatas), Al₂O₃ (Tonerde), Aluminiumsilikat, Steatit (Magnesiumsilikat), Zirkoniumsilikat, Cersilikat oder Mischungen dieser Trägermaterialien, eingesetzt werden. Bevorzugte Trägermaterialien sind Aluminiumoxid (Al₂O₃) und Siliziumdioxid (SiO₂), ganz besonders Aluminiumoxid.
In der bevorzugten Ausführungsform des erfindungsgemäßen Verfahrens verwendet man Kupferkatalysatoren, die in der DE 2 445 303 A1 (BASF AG) beschrieben worden sind. Sie können als amorphe Produkte der thermischen Zersetzung und Reduktion basischer Kupfer-Aluminium-Carbonate angesehen und dadurch erhalten werden, dass man verdünnte oder mäßig konzentrierte, zweckmäßig unter 3-molare Lösungen von Kupfer- und Aluminiumsalzen mit Alkalicarbonat bei pH 8 - 10 fällt und die erhaltenen Niederschläge vor oder nach entsprechender Formung bei einer Temperatur von 350 - 600 °C zersetzt. Nach üblicher Reduzierung, bevorzugt in Gegenwart des bei der späteren Umsetzung verwendeten Alkohols, erhält man hochaktive, für das vorliegende Verfahren bestens geeignete Katalysatoren.
Ein Beispiel für einen geeigneten Katalysatoren wie DE 24 45 303 A offenbart, erhalten durch Temperung eines basischen Kupfer und Aluminium enthaltenen Carbonats der allgemeinen Zusammensetzung CuₘAl₆(CO₃)_{0,5m}O₃(OH)ₘ₊₁₂, wobei m einen beliebigen, auch nicht ganzzahligen, Wert zwischen 2 und 6 bedeutet, ist der in loc. cit., Beispiel 1, offenbarte kupferhaltige Fällkatalysator, der durch Behandlung einer Lösung von Kupfernitrat und Aluminiumnitrat mit Natriumbicarbonat und anschließendem Waschen, Trocknen und Tempern des Präzipitats hergestellt wird.

Bei der nach dem erfindungsgemäßen Verfahren ebenfalls möglichen Suspensionsfahrweise ist der reduzierte Kupferkatalysator in der Reaktionskomponente Diethanolamin suspendiert. Geeignete Katalysatoren sind z. B. Raney-Kupfer oder die oben beschriebenen Kupferkatalysatoren in gepulverter Form. Bevorzugt ist jedoch ein kupferhaltiges Material, das man dadurch erhält, dass man Kupferformiat in Gegenwart eines Alkohols und Dialkylamin auf 200 - 250 °C erhitzt. Die Bildungsweise eines solchen Katalysators wird z. B. in der EP 70 512 A beschrieben.

Die Katalysatoren können als Formkörper eingesetzt werden, z. B. in Form von Kugeln, Ringen, Zylindern, Würfeln, Quadern oder anderen geometrischen Körpern. Ungeträgerte Katalysatoren können nach üblichen Verfahren geformt werden, z. B. durch Extrudieren, Tablettieren, etc. Die Form geträgerter Katalysatoren wird durch die Form des Trägers bestimmt. Alternativ dazu kann der Träger vor oder nach dem Aufbringen der katalytisch aktiven Komponente(n) einem Formungsverfahren unterzogen werden. Die Katalysatoren können z. B. in Form von gepressten Zylindern, Tabletten, Pastillen, Wagenrädern, Ringen, Sternen oder Strangpresslingen, wie Vollsträngen, polylobären Strängen, Hohlsträngen und Wabenkörpern oder anderen geometrischen Körpern, eingesetzt werden.

Im erfindungsgemäßen Verfahren werden die Katalysatoren besonders bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.
In diesem Zusammenhang wird oxidisches Trägermaterial, besonders bevorzugt Aluminiumoxid (Al₂O₃), als zur katalytisch aktiven Masse gehörig gewertet.
Die Katalysatoren werden bevorzugt dergestalt eingesetzt, dass man die katalytisch aktive Masse nach Mahlung, Vermischung mit Formhilfsmitteln, Formung und Temperung als Katalysatorformkörper - z.B. als Tabletten, Kugeln, Ringe, Extrudate (z.B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und des o. g. Katalysatorträgermaterials definiert und enthält bevorzugt im wesentlichen die folgenden Bestandteile:
Aluminiumoxid (Al₂O₃) und sauerstoffhaltige Verbindungen des Kupfers und bevorzugt sauerstoffhaltige Verbindungen des Natriums.

Die Summe der o. g. Bestandteile der katalytisch aktiven Masse, berechnet als Al₂O₃, CuO und Na₂O, beträgt üblicherweise 70 bis 100 Gew.-%, bevorzugt 80 bis 100 Gew.-%, besonders bevorzugt 90 bis 100 Gew.-%, weiter bevorzugt 98 bis 100 Gew.-%, weiter bevorzugt ≥ 99 Gew.-%, ganz besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:

Übergangsmetalle, wie Ni bzw. NiO, Co bzw. CoO, Re bzw. Rheniumoxide, Mn bzw. MnO₂, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Alkalimetalloxide, wie K₂O; Alkalimetallcarbonate, wie Na₂CO₃; Erdalkalimetalloxide, wie CaO, SrO; Erdalkalimetallcarbonate, wie MgCO₃, CaCO₃ und BaCO₃; Boroxid (B₂O₃).

Die katalytisch aktive Masse der im erfindungsgemäßen Verfahren eingesetzten Katalysatoren enthält nach deren letzter Wärmebehandlung und vor deren Reduktion mit Wasserstoff besonders
20 bis 80 Gew.-%, bevorzugt 30 bis 70 Gew.-%, besonders bevorzugt 35 bis 60 Gew.-%, Aluminiumoxid (Al₂O₃) und
20 bis 80 Gew.-%, bevorzugt 30 bis 70 Gew.-%, besonders bevorzugt 40 bis 65 Gew.-%, ganz besonders bevorzugt 45 bis 60 Gew.-%, sauerstoffhaltige Verbindungen des Kupfers, berechnet als CuO,
0 bis 2 Gew.-%, bevorzugt 0,05 bis 1 Gew.-%, besonders bevorzugt 0,1 bis 0,5 Gew.-%, sauerstoffhaltige Verbindungen des Natriums, berechnet als Na₂O,
weniger als 5 Gew.-%, z. B. 0,1 bis 4 Gew.-%, bevorzugt weniger als 1 Gew.-%, z. B. 0 bis 0,8 Gew.-%, sauerstoffhaltige Verbindungen des Nickels, berechnet als NiO.

Die katalytisch aktive Masse des Katalysators enthält vor dessen Reduktion mit Wasserstoff besonders bevorzugt weniger als 1 Gew.-%, z. B. 0 bis 0,5 Gew.-%, sauerstoffhaltige Verbindungen des Kobalts, berechnet als CoO.

Ganz besonders bevorzugt enthält die katalytisch aktive Masse des im erfindungsgemäßen Verfahren eingesetzten Katalysators kein Nickel, kein Kobalt und/oder kein Ruthenium, jeweils weder in metallischer (Oxidationsstufe 0) noch in einer ionischen, insb. oxidierten, Form.

Bei den sauerstoffhaltigen Verbindungen des Kupfers handelt es sich insbesondere um Kupfer-(I)-oxid und Kupfer-(II)-oxid, bevorzugt um Kupfer-(II)-oxid.

Ganz besonders bevorzugt enthält die katalytisch aktive Masse des im erfindungsgemäßen Verfahren eingesetzten Katalysators kein Zirkoniumdioxid (ZrO₂), Titandioxid (TiO₂) und/oder Siliziumdioxid (SiO₂).

In einer besonders bevorzugten Ausführungsform enthält die katalytisch aktive Masse der im erfindungsgemäßen Verfahren eingesetzten Katalysatoren keine weitere katalytisch aktive Komponente, weder in elementarer noch in ionischer Form.
In der besonders bevorzugten Ausführungsform ist die katalytisch aktive Masse nicht mit weiteren Metallen oder Metallverbindungen dotiert.
Bevorzugt sind jedoch aus der Metallgewinnung von Cu, ggf. Ni, herrührende übliche Begleit-Spurenelemente hiervon ausgenommen.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten Aluminium, Kupfer, ggf. Natrium mit Wasser und nachfolgendes Extrudieren und Tempern der so erhaltenen Masse erhältlich.

Die im erfindungsgemäßen Verfahren bevorzugt verwendeten Katalysatoren können auch durch Tränkung von Aluminiumoxid (Al₂O₃), das beispielsweise in Form von Pulver oder Tabletten-Formkörpem vorliegt, hergestellt werden.

Aluminiumoxid kann dabei in verschiedenen Modifikationen eingesetzt werden, bevorzugt sind α- (alpha), γ-(gamma) oder θ-Al₂O₃ (theta-Al₂O₃). Besonders bevorzugt wird γ-Al₂O₃ eingesetzt.

Die Herstellung von Formkörpern des Aluminiumoxids kann nach den üblichen Verfahren erfolgen.

Die Tränkung des Aluminiumoxids erfolgt ebenfalls nach den üblichen Verfahren, wie z. B. in EP 599 180 A, EP 673 918 A oder A. B. Stiles, Catalyst Manufacture -Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und ggf. calciniert.

Die Tränkung kann nach der sogenannten "incipient wetness"-Methode erfolgen, bei der das anorganische Oxid (d. h. Aluminiumoxid) entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft besonders dann anzuwenden, wenn das anorganische Oxid mit einer größeren Metallmenge beaufschlagt werden soll.

Zur Aufbringung mehrerer Metallkomponenten auf das anorganische Oxid kann die Tränkung gleichzeitig mit ggf. allen Metallsalzen oder in beliebiger Reihenfolge der ggf. einzelnen Metallsalze nacheinander erfolgen.

Bevorzugt werden zur Herstellung der im erfindungsgemäßen Verfahren bevorzugt verwendeten Katalysatoren Fällungsmethoden angewandt. So können sie beispielsweise durch eine gemeinsame Fällung der Komponenten aus einer wässrigen Salzlösung mittels Mineralbasen in Gegenwart einer Aufschlämmung einer schwerlöslichen, sauerstoffhaltigen AluminiumVerbindung und anschließendes Waschen, Trocknen und Calcinieren des erhaltenen Präzipitats erhalten werden. Als schwerlösliche, sauerstoffhaltige Aluminiumverbindung kann beispielsweise Aluminiumoxid Verwendung finden. Die Aufschlämmungen der schwerlöslichen Aluminiumverbindung kann durch Suspendieren feinkörniger Pulver dieser Verbindung in Wasser unter kräftigem Rühren hergestellt werden. Vorteilhaft werden diese Aufschlämmungen durch Ausfällen der schwerlöslichen Aluminiumverbindung aus wässrigen Aluminium-Salzlösungen mittels Mineralbasen erhalten.

Bevorzugt werden die im erfindungsgemäßen Verfahren bevorzugt verwendeten Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre, zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze des eingesetzten Metalls/der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden wie üblich zu den erfindungsgemäß verwendeten Katalysatoren weiterverarbeitet. Nach dem Waschen werden sie im bevorzugt bei 80 bis 200 °C, vorzugsweise bei 100 bis 150 °C, getrocknet und danach calciniert. Die Calcinierung wird bevorzugt bei Temperaturen zwischen 300 und 800 °C, vorzugsweise 400 bis 600 °C, insbesondere 450 bis 550 °C, ausgeführt.

Nach der Calcinierung wird der Katalysator zweckmäßigerweise konditioniert, sei es, dass man ihn durch Vermahlen auf eine bestimmte Korngröße einstellt und/oder dass man ihn nach seiner Vermahlung mit Formhilfsmitteln wie Graphit oder Stearinsäure vermischt, mittels einer Presse zu den Formlingen, nämlich Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren können als solche gelagert werden. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200 °C über einen Zeitraum von z. B. 12 bis 20 Stunden einer Stickstoff-Wasserstoff-Atmosphäre ausgesetzt und anschließend noch bis zu ca. 24 Stunden bei bevorzugt 200 bis 400 °C in einer Wasserstoffatmosphäre behandelt. Bei dieser Vorreduktion wird ein Teil der in den Katalysatoren vorliegenden sauerstoffhaltigen Metallverbindung/en zu dem/den entsprechenden Metall/en reduziert, so dass diese gemeinsam mit den verschiedenartigen Sauerstoffverbindungen in der aktiven Form des Katalysators vorliegen.

Die Umsetzung gemäß erfindungsgemäßem Verfahren erfolgt bevorzugt in einem Rohrreaktor. Bei einer Monostranganlage besteht der Rohrreaktor, in dem die bevorzugt isotherme Umsetzung erfolgt, aus einer Hintereinanderschaltung mehrerer (z. B. zweier oder dreier) einzelner Rohrreaktoren.

Die bevorzugt isotherme Umsetzung gemäß dem erfindungsgemäßen Verfahren erfolgt bevorzugt mit einer Temperaturabweichung von maximal +/- 8 °C, besonders maximal +/- 5 °C, insbesondere maximal +/- 4 °C, ganz besonders maximal +/- 3 °C, z. B. maximal +/- 0 bis +/- 2 °C oder maximal +/- 0 bis +/- 1 °C.

Diese Temperaturabweichungen beziehen sich auf die jeweiligen Temperaturen im jeweiligen Katalysatorbett, und zwar beim Eintritt der Edukte in das Katalysatorbett und beim Austritt der Reaktionsmischung aus dem Katalysatorbett.
Dabei können mehrere Katalysatorbetten parallel oder in Reihe ge- oder verschaltet sein.

Sind mehrere Katalysatorbetten in Reihe geschaltet, beziehen sich die genannten Temperaturabweichungen bei der erfindungsgemäß bevorzugten isothermen Fahrweise auf die jeweilige Temperatur im Katalysatorbett, und zwar beim Eintritt der Edukte in das erste Katalysatorbett und beim Austritt der Reaktionsmischung aus dem letzten Katalysatorbett.

In einer bevorzugten Ausführungsform erfolgt die Temperierung des Reaktorrohrs von außen mit einem Wärmeträgerstrom, wobei es sich bei dem Wärmeträger z. B. um ein Öl, eine Salzschmelze oder eine andere wärmeübertragende Flüssigkeit handeln kann.

Das erfindungsgemäße Verfahren wird bevorzugt kontinuierlich durchgeführt, wobei der Katalysator bevorzugt als Festbett im Reaktor angeordnet ist. Dabei ist sowohl eine Anströmung des Katalysatorfestbetts von oben als auch von unten möglich.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Eine weitere bevorzugte Ausführungsform ist, mit Hilfe eines Lösungsmittels, das ein Heteroazeotrop mit Wasser bildet (z. B. Benzol, Toluol, Xylol), das Reaktionswasser durch Azeotropdestillation zu entfernen. Das Destillat der Azeotropdestillation bildet nach Kühlung und Entspannung ein zweiphasiges flüssiges Gemisch. Nach Phasentrennung wird Wasser ausgeschleust und die organische Phase (also das Lösungsmittel) in den Aufarbeitungsschritt zurückgeführt.

Der nach Kühlung und Entspannung anfallende flüssige Reaktionsaustrag wird gegebenenfalls vom suspendierten Katalysator abgetrennt. Der katalysatorfreie Reaktionsaustrag enthält neben dem Zielprodukt, dem tertiären Amin I, gegebenenfalls noch unumgesetztes Diethanolamin II und gebildetes Wasser. Es entstehen zwei Mole Wasser, pro Mol eingesetztes Diethanolamin.

Der Reaktionsaustrag kann destillativ aufgearbeitet werden. Dabei werden Reaktionswasser, gegebenenfalls Lösungsmittel und ggf. nicht umgesetztes Diethanolamin II zunächst über Kopf abgetrennt. Das überwiegend aus BHEPIP (I) bestehende Sumpfprodukt kann durch weitere fraktionierende Destillation gereinigt werden. Nicht umgesetztes Diethanolamin II kann in die Synthesestufe zurückgeführt werden.

1,4-Bishydroxyethylpiperazin I besitzt einen Schmelzpunkt von 135 °C. Es ist daher auch möglich, I in kristalliner Form z. B. durch Filtration oder Zentrifugieren abzutrennen,

Bei der Dimerisierung von Diethanolamin II kann die Zwischenstufe III durchlaufen werden:

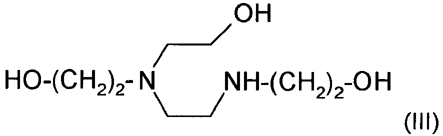

Alle Druckangaben beziehen sich auf den Absolutdruck.

### Beispiele

Für die folgenden Beispiele wurde ein Kupfer-Katalysator der Zusammensetzung 55 Gew.-% CuO und 45 Gew.-% gamma-Al₂O₃ (nach dessen letzter Wärmebehandlung und vor dessen Reduktion mit Wasserstoff) verwendet (Katalysator A).
Die Herstellung des Katalysators erfolgte durch Tränkung von gamma-Al₂O₃ - Pulver mit einer wässrigen Kupfernitrat-Lösung. Die Tablettierung erfolgte nach üblicher Methode. Vor Beginn der Umsetzung wurde der Katalysator im Wasserstoffstrom bei ca. 200 °C reduziert (s.u.).
Die Angaben zur Ausbeute an BHEPIP (I) stellen gaschromatographisch ermittelte Flächen-%-Werte dar.

### Beispiel 1

### Herstellung von 1,4-Bis(hydroxyethyl)piperazin I aus Diethanolamin (Lösungsmittel Tetrahydrofuran)

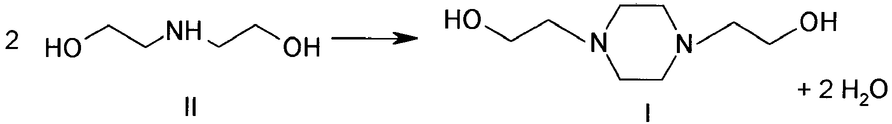

Die Umsetzung wurde in einem magnetgekuppelten 300 ml Rührautoklaven mit Elektroheizung und Innentemperatur-Kaskadenregelung durchgeführt.

In den mit Stickstoff inertisierten Autoklaven wurden 31,5 g Diethanolamin (II) (0,3 mol), 50 g Tetrahydrofuran (THF) und 10 g des reduzierten und passivierten, Kupfer auf Aluminiumoxid enthaltenden Katalysators A (3 x 3 mm Tabletten) eingefüllt. Der Katalysator bestand vor der Reduktion zu 55 Gew.-% aus Kupferoxid (CuO) und zu 45 Gew.-% aus Aluminiumoxid. Die Reduktion war vor der Umsetzung bei 180 bis 200 °C, die Passivierung bei < 50 °C mit Luft erfolgt. Auf das Reaktionsgemisch wurde bei Raumtemperatur Wasserstoff bis zu einem Druck von 10 bar aufgepresst. Dann wurde auf 200 °C erhitzt, Wasserstoff bis zu einem Gesamtdruck von 80 bar nachgepresst und 10 Stunden lang bei 200 °C und 80 bar gerührt (700 rpm).

Die gaschromatographische Analyse (GC-Säule: 30 m RTX 5 Amin) ergab, dass der Reaktionsaustrag bei vollständigem Diethanolamin-Umsatz zu 73 % aus 1,4-Bis(hydroxyethyl)-piperazin bestand (THF herausgerechnet).

### Beispiel 2

### Herstellung von 1,4-Bis(hydroxyethyl)piperazin aus Diethanolamin (kein Lösungsmittel)

Beispiel 2 wurde unter den Bedingungen von Beispiel 1 in der selben Apparatur durchgeführt. Dabei wurden 100 g Diethanolamin (II) und 10 g Katalysator A eingesetzt. Die gaschromatographische Analyse (GC-Säule: 30 m RTX Amin) ergab bei vollständigem Diethanolamin-Umsatz, dass der Reaktionsaustrag zu 79 % aus 1,4-Bis(hydroxyethyl)piperazin bestand.

Das Beispiel zeigt, dass erfindungsgemäß 1,4-Bis(hydroxyethyl)piperazin I auch ohne Verwendung eines Lösungsmittels, hier in Festbettfahrweise, in hoher Ausbeute herstellbar ist.

## Patentansprüche

1. Verfahren zur Herstellung von 1,4-Bishydroxyethyl-piperazin (BHEPIP) der Formel I 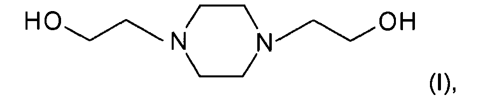 **dadurch gekennzeichnet, dass** man Diethanolamin (DEOA) der Formel II 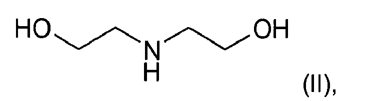 in einem Reaktor bei einer Temperatur im Bereich von 130 bis 300 °C in Gegenwart eines kupferhaltigen, chromfreien Heterogenkatalysators in der Flüssigphase umsetzt.

2. Verfahren nach dem vorhergehenden Anspruch, **dadurch gekennzeichnet, dass** man die Umsetzung in Gegenwart eines kupfer- und aluminiumoxidhaltigen Katalysators durchführt.

3. Verfahren nach einem der beiden vorhergehenden Ansprüche, **dadurch gekennzeichnet, dass** die katalytisch aktive Masse des Katalysators vor dessen Reduktion mit Wasserstoff
20 bis 80 Gew.-% Aluminiumoxid (Al₂O₃),
20 bis 80 Gew.-% sauerstoffhaltige Verbindungen des Kupfers, berechnet als CuO,
0 bis 2 Gew.-% sauerstoffhaltige Verbindungen des Natriums, berechnet als Na₂O, und weniger als 5 Gew.-% sauerstoffhaltige Verbindungen des Nickels, berechnet als NiO, enthält.

4. Verfahren nach einem der vorhergehenden Ansprüche, **dadurch gekennzeichnet, dass** die katalytisch aktive Masse des Katalysators vor dessen Reduktion mit Wasserstoff weniger als 1 Gew.-% sauerstoffhaltige Verbindungen des Nickels, berechnet als NiO, enthält.

5. Verfahren nach einem der vorhergehenden Ansprüche, **dadurch gekennzeichnet, dass** die katalytisch aktive Masse des Katalysators vor dessen Reduktion mit Wasserstoff weniger als 1 Gew.-% sauerstoffhaltige Verbindungen des Kobalts, berechnet als CoO, enthält.

6. Verfahren nach einem der vorhergehenden Ansprüche, **dadurch gekennzeichnet, dass** die katalytisch aktive Masse des Katalysators vor dessen Reduktion mit Wasserstoff
30 bis 70 Gew.-% Aluminiumoxid (Al₂O₃) und
30 bis 70 Gew.-% sauerstoffhaltige Verbindungen des Kupfers, berechnet als CuO, enthält.

7. Verfahren nach einem der vorhergehenden Ansprüche, **dadurch gekennzeichnet, dass** die katalytisch aktive Masse des Katalysators vor dessen Reduktion mit Wasserstoff 0,05 bis 1 Gew.-% sauerstoffhaltige Verbindungen des Natriums, berechnet als Na₂O, enthält.

8. Verfahren nach einem der vorhergehenden Ansprüche, **dadurch gekennzeichnet, dass** die katalytisch aktive Masse des Katalysators kein Nickel, Kobalt und/oder Ruthenium enthält.

9. Verfahren nach einem der vorhergehenden Ansprüche, **dadurch gekennzeichnet, dass** die Umsetzung isotherm, mit einer Temperaturabweichung von maximal +/- 8 °C erfolgt.

10. Verfahren nach einem der vorhergehenden Ansprüche, **dadurch gekennzeichnet, dass** man die Umsetzung in Gegenwart von Wasserstoff (H₂) durchführt.

11. Verfahren nach einem der vorhergehenden Ansprüche, **dadurch gekennzeichnet, dass** die Umsetzung kontinuierlich erfolgt.

12. Verfahren nach dem vorhergehenden Anspruch, **dadurch gekennzeichnet, dass** die Umsetzung in einem Rohrreaktor erfolgt.

13. Verfahren nach einem der beiden vorhergehenden Ansprüche, **dadurch gekennzeichnet, dass** die Umsetzung in einem Rohrbündelreaktor oder in einer Monostranganlage erfolgt.

14. Verfahren nach einem der vorhergehenden Ansprüche, **dadurch gekennzeichnet, dass** man die Umsetzung bei einem Absolutdruck im Bereich von 10 bis 200 bar durchführt.

15. Verfahren nach einem der vorhergehenden Ansprüche, **dadurch gekennzeichnet, dass** der Katalysator im Reaktor als Festbett angeordnet ist.

16. Verfahren nach einem der vorhergehenden Ansprüche, **dadurch gekennzeichnet, dass** man die Umsetzung in Abwesenheit eines Lösungsmittels durchführt.

## Claims

1. A process for preparing 1,4-bishydroxyethylpiperazine (BHEPIP) of the formula I 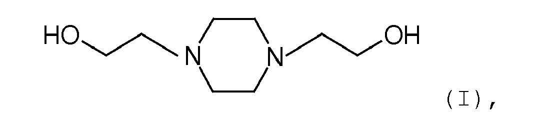 wherein diethanolamine (DEOA) of the formula II 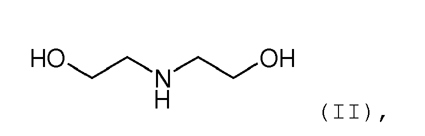 is reacted in the liquid phase in a reactor at a temperature in the range from 130 to 300°C in the presence of a copper-comprising, chromium-free heterogeneous catalyst.

2. The process according to the preceding claim, wherein the reaction is carried out in the presence of a copper- and aluminum oxide-comprising catalyst.

3. The process according to either of the two preceding claims, wherein the catalytically active composition of the catalyst before reduction with hydrogen comprises from 20 to 80% by weight of aluminum oxide (Al₂O₃), from 20 to 80% by weight of oxygen-comprising compounds of copper, calculated as CuO, from 0 to 2% by weight of oxygen-comprising compounds of sodium, calculated as Na₂O, and less than 5% by weight of oxygen-comprising compounds of nickel, calculated as NiO.

4. The process according to any of the preceding claims, wherein the catalytically active composition of the catalyst before reduction with hydrogen comprises less than 1% by weight of oxygen-comprising compounds of nickel, calculated as NiO.

5. The process according to any of the preceding claims, wherein the catalytically active composition of the catalyst before reduction with hydrogen comprises less than 1% by weight of oxygen-comprising compounds of cobalt, calculated as CoO.

6. The process according to any of the preceding claims, wherein the catalytically active composition of the catalyst before reduction with hydrogen comprises from 30 to 70% by weight of aluminum oxide (Al₂O₃) and from 30 to 70% by weight of oxygen-comprising compounds of copper, calculated as CuO.

7. The process according to any of the preceding claims, wherein the catalytically active composition of the catalyst before reduction with hydrogen comprises from 0.05 to 1% by weight of oxygen-comprising compounds of sodium, calculated as Na₂O.

8. The process according to any of the preceding claims, wherein the catalytically active composition of the catalyst does not comprise any nickel, cobalt and/or ruthenium.

9. The process according to any of the preceding claims, wherein the reaction is carried out isothermally with a temperature deviation of not more than +/- 8°C.

10. The process according to any of the preceding claims, wherein the reaction is carried out in the presence of hydrogen (H₂).

11. The process according to any of the preceding claims, wherein the reaction is carried out continuously.

12. The process according to the preceding claim, wherein the reaction is carried out in a tube reactor.

13. The process according to either of the two preceding claims, wherein the reaction is carried out in a shell-and-tube reactor or in a single-train plant.

14. The process according to any of the preceding claims, wherein the reaction is carried out at an absolute pressure in the range from 10 to 200 bar.

15. The process according to any of the preceding claims, wherein the catalyst is arranged as a fixed bed in the reactor.

16. The process according to any of the preceding claims, wherein the reaction is carried out in the absence of a solvent.

## Revendications

1. Procédé de fabrication de 1,4-bishydroxyéthylpipérazine (BHEPIP) de formule I 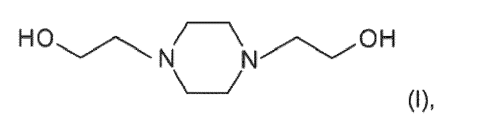 **caractérisé en ce que** de la diéthanolamine (DEOA) de formule II 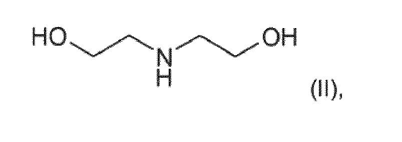 est mise en réaction en phase liquide dans un réacteur à une température dans la plage allant de 130 à 300 °C en présence d'un catalyseur hétérogène sans chrome, contenant du cuivre.

2. Procédé selon la revendication précédente, **caractérisé en ce que** la réaction est réalisée en présence d'un catalyseur contenant de l'oxyde de cuivre et d'aluminium.

3. Procédé selon l'une quelconque des deux revendications précédentes, **caractérisé en ce que** la masse catalytiquement active du catalyseur contient avant sa réduction avec de l'hydrogène
20 à 80 % en poids d'oxyde d'aluminium (Al₂O₃),
20 à 80 % en poids de composés oxygénés de cuivre, calculés en tant que CuO,
0 à 2 % en poids de composés oxygénés de sodium, calculés en tant que Na₂O, et
moins de 5 % en poids de composés oxygénés de nickel, calculés en tant que NiO.

4. Procédé selon l'une quelconque des revendications précédentes, **caractérisé en ce que** la masse catalytiquement active du catalyseur contient avant sa réduction avec de l'hydrogène moins de 1 % en poids de composés oxygénés de nickel, calculés en tant que NiO.

5. Procédé selon l'une quelconque des revendications précédentes, **caractérisé en ce que** la masse catalytiquement active du catalyseur contient avant sa réduction avec de l'hydrogène moins de 1 % en poids de composés oxygénés de cobalt, calculés en tant que CoO.

6. Procédé selon l'une quelconque des revendications précédentes, **caractérisé en ce que** la masse catalytiquement active du catalyseur contient avant sa réduction avec de l'hydrogène
30 à 70 % en poids d'oxyde d'aluminium (Al₂O₃,) et
30 à 70 % en poids de composés oxygénés de cuivre, calculés en tant que CuO.

7. Procédé selon l'une quelconque des revendications précédentes, **caractérisé en ce que** la masse catalytiquement active du catalyseur contient avant sa réduction avec de l'hydrogène 0,05 à 1 % en poids de composés oxygénés de sodium, calculés en tant que Na₂O.

8. Procédé selon l'une quelconque des revendications précédentes, **caractérisé en ce que** la masse catalytiquement active du catalyseur ne contient pas de nickel, de cobalt et/ou de ruthénium.

9. Procédé selon l'une quelconque des revendications précédentes, **caractérisé en ce que** la réaction a lieu de manière isotherme, avec une déviation de température d'au plus ± 8 °C.

10. Procédé selon l'une quelconque des revendications précédentes, **caractérisé en ce que** la réaction est réalisée en présence d'hydrogène (H₂).

11. Procédé selon l'une quelconque des revendications précédentes, **caractérisé en ce que** la réaction a lieu en continu.

12. Procédé selon la revendication précédente, **caractérisé en ce que** la réaction a lieu dans un réacteur tubulaire.

13. Procédé selon l'une quelconque des deux revendications précédentes, **caractérisé en ce que** la réaction a lieu dans un réacteur à faisceau de tubes ou dans une unité à gaine unique.

14. Procédé selon l'une quelconque des revendications précédentes, **caractérisé en ce que** la réaction est réalisée à une pression absolue dans la plage allant de 10 à 200 bar.

15. Procédé selon l'une quelconque des revendications précédentes, **caractérisé en ce que** le catalyseur est agencé dans le réacteur sous la forme d'un lit fixe.

16. Procédé selon l'une quelconque des revendications précédentes, **caractérisé en ce que** la réaction est réalisée en l'absence d'un solvant.
